# EUROPEAN PATENT APPLICATION

(11) **EP 4 692 373 A1**
(43) Date of publication of application: **11.02.2026**
(21) Application number: 24778016.6
(22) Date of filing: 26.03.2024
(51) Int. Cl.: C12Q 1/6886, C12Q 1/68, G01N 33/00

(54) **USE OF DETECTING METHYLATION LEVEL OF TXNRD1 GENE IN DIAGNOSIS AND/OR SCREENING OF ENDOMETRIAL CANCER**

(30) Priority: 29.03.2023 CN 202310319888
(71) Applicant: Beijing Youxun Yicheng Medical Equipment Co., Ltd., Beijing (CN)
(72) Inventor: ZHAO, Hanqing, Beijing 100195 (CN); LIU, Yunchao, Beijing 100195 (CN); CHANG, Luyuan, Beijing 100195 (CN); WU, Qixi, Beijing 100195 (CN); WANG, Jianwei, Beijing 100195 (CN)
(74) Representative: Winter, Brandl - Partnerschaft mbB
(86) International application number: PCT/CN2024/083844
(87) International publication number: WO 2024/199228

(57) **Abstract**

The present application relates to the technical field of molecular biology detection, and in particular, to the use of detecting the methylation level of the TXNRD1 gene in the diagnosis and/or screening of endometrial cancer. Provided are a kit for the diagnosis and/or screening of endometrial cancer with cervical exfoliated cells as detection samples, and the use thereof, wherein the TXNRD1 gene is used as a target, and the detection of TXNRD1 gene methylation can be used in the diagnosis and/or screening of endometrial cancer. A cervical swab sample can be collected at an outpatient service without the need for invasive sampling, such that same has higher patient acceptance.

## Description

The present application is based upon and claims the benefit of priority from Chinese Application No. 202310319888.4, filed on 29 March 2023, the disclosure of which is incorporated herein by reference in its entirety.

### Technical Field

The present application generally relates to the technical field of molecular biology detection, and specifically relates to use of methylation level detection of TXNRD1 gene in the diagnosis and/or screening of endometrial cancer.

### Background

Endometrial cancer refers to a group of epithelial malignant tumors occurring in the endometrium, also termed corpus carcinoma, and is one of the three common malignant tumors of the female genital tract. Endometrial cancer is the most common malignant tumor of the female reproductive system in developed countries, and in recent years, the incidence has shown an increasing trend and the affected population is becoming younger. Current methods for screening endometrial cancer include transvaginal ultrasonography, endometrial microtissue pathology, and endometrial cytology. Transvaginal ultrasonography has limited sensitivity to premenopausal women, and is not recommended as a method for individually screening endometrial cancer. Endometrial microtissue pathology and endometrial cytology, although they can be performed without anesthesia or cervical dilation, require invasive access to the uterine cavity to obtain endometrial tissue or cells, which leads to low satisfaction with the sample collection and can easily cause discomfort to patients. Furthermore, both methods lack strict diagnostic criteria unanimously recognized by pathologists or cytologists for the diagnosis of pathological tissue and interpretation of cell smears, resulting in a high degree of subjectivity. In addition, as the changes in the endometrium are related to the levels of hormones, the sampling time of the cytology will also affect the determination of results. There is an urgent need to develop a new screening method for endometrial cancer, which can be used without invasive or minimally invasive sampling and objectively evaluate results and has higher sensitivity and specificity.

DNA methylation is a chemical modification process in which a specific base in a DNA sequence obtains a methyl group through covalent binding under the catalysis of DNA methyltransferase (DNMT) using S-adenosylmethionine (SAM) as a methyl donor. DNA methylation occurs mainly on C of 5'-CpG-3' to generate 5-methylcytosine (5 mC). In normal tissues, 70%-90% of dispersed CpG is modified by methyl, which has an important effect on maintaining the stability of gene families, while CpG located in a gene transcription regulation region, such as a CpG island in a promoter region, can regulate gene transcription through methylation. Studies have shown that DNA in the promoter region is generally capable of inhibiting gene expression after methylation, resulting in gene silencing. However, hypermethylation of the promoter region causes the silencing of tumor suppressor genes and is therefore closely related to the occurrence of tumors, and this phenomenon has been shown in the early stage of tumorigenesis, and therefore DNA methylation is considered to be an excellent potential biomarker for screening in the early stage of tumors.

In view of this, the present application is specially proposed for the diagnosis and/or screening of endometrial cancer.

### Summary

The present application relates to a primer-probe combination product, comprising at least one of the following combinations:
a) primers having nucleotide sequences as shown in SEQ ID NOs: 1-2 and a probe having a nucleotide sequence as shown in SEQ ID NO: 3;
b) primers having nucleotide sequences as shown in SEQ ID NOs: 4-5 and a probe having a nucleotide sequence as shown in SEQ ID NO: 6;
c) primers having nucleotide sequences as shown in SEQ ID NOs: 7-8 and a probe having a nucleotide sequence as shown in SEQ ID NO: 9;
d) primers having nucleotide sequences as shown in SEQ ID NOs: 10-11 and a probe having a nucleotide sequence as shown in SEQ ID NO: 12;
e) primers having nucleotide sequences as shown in SEQ ID NOs: 13-14 and a probe having a nucleotide sequence as shown in SEQ ID NO: 15; and
f) primers having nucleotide sequences as shown in SEQ ID NOs: 16-17 and a probe having a nucleotide sequence as shown in SEQ ID NO: 18.

According to a further aspect of the present application, provided is a kit comprising the described primer-probe combination product.

According to a further aspect of the present application, provided is use of a detection agent for the methylation level of TXNRD1 gene in the preparation of an endometrial cancer diagnosis and/or screening reagent or kit.

According to another aspect of the present application, provided is a method for diagnosing and/or screening endometrial cancer. The described method contains: diagnosing and/or screening endometrial cancer by detecting the methylation level of TXNRD1 gene.

Further, detecting the methylation level of the TXNRD1 gene contains detecting the methylation level of a chr12:104215491-104216453 region of the TXNRD1 gene.

Further, detecting the methylation level of the TXNRD1 gene contains detecting the methylation level of at least one of the following regions: chr12:104215491-104215646, chr12:104215647-104215765, chr12:104215750-104215872, chr12:104215852-104216065, chr12:104216066-104216200, and chr12:104216190-104216453.

Further, the detection contains any one or more of the following methods: methylation-specific PCR, pyrosequencing or bisulfite sequencing, methylation-specific microarray, methylation-specific high performance liquid chromatography, high resolution melting (HRM) analysis, methylation-sensitive restriction endonuclease or fluorescence quantitation, methylation-specific denaturation high performance liquid chromatography, HPLC-UV, LC-MS/MS, methylation-sensitive single-strand conformation analysis, methylation-sensitive denaturation gradient gel electrophoresis, methylation-sensitive melting curve analysis, Methylight technology, methylation-sensitive dot blot analysis, enrichment methods of methylated antibody or MBD affinity, MeDIP-Seq, RRBS-Seq, WGBS, MBD-Seq, and SMRT.

Further, the detection agent for detection contains the primer-probe combination product of any one of claims 1-7.

Further, the detection sample in the method is a cervical exfoliated tissue or cell or a lysate thereof of a subject.

According to another aspect of the present application, provided is a primer-probe combination for diagnosing and/or screening endometrial cancer. The described primer-probe combination is used for detecting the methylation level of the TXNRD1 gene.

Further, detecting the methylation level of the TXNRD1 gene contains detecting the methylation level of a chr12:104215491-104216453 region of the TXNRD1 gene.

Further, detecting the methylation level of the TXNRD1 gene contains detecting the methylation level of at least one of the following regions: chr12:104215491-104215646, chr12:104215647-104215765, chr12:104215750-104215872, chr12:104215852-104216065, chr12:104216066-104216200, and chr12:104216190-104216453.

Further, the detection contains any one or more of the following methods: methylation-specific PCR, pyrosequencing or bisulfite sequencing, methylation-specific microarray, methylation-specific high performance liquid chromatography, high resolution melting (HRM) analysis, methylation-sensitive restriction endonuclease or fluorescence quantitation, methylation-specific denaturation high performance liquid chromatography, HPLC-UV, LC-MS/MS, methylation-sensitive single-strand conformation analysis, methylation-sensitive denaturation gradient gel electrophoresis, methylation-sensitive melting curve analysis, Methylight technology, methylation-sensitive dot blot analysis, enrichment methods of methylated antibody or MBD affinity, MeDIP-Seq, RRBS-Seq, WGBS, MBD-Seq, and SMRT.

Further, the primer-probe combination is the primer-probe combination product of any one of claims 1-7.

Further, the detection sample used for the detection is a cervical exfoliated tissue or cell or a lysate thereof of a subject.

The present application provides a kit for diagnosing and/or screening endometrial cancer using a cervical exfoliated cell as a detection sample and use. The kit uses the TXNRD1 gene as a target, and can be used for diagnosing and/or screening endometrial cancer by detecting methylation thereof. A cervical swab sample can be sampled in an outpatient clinic without invasive sampling, has a higher patient acceptance, and has advantages such as a fast detection speed and higher sensitivity and specificity, which makes the primary screening work of endometrial cancer more convenient and popularized, and has important significance for early diagnosis and treatment of endometrial cancer.

### Brief Description of the Drawings

To describe the technical solutions in particular embodiments of the present application or in the prior art more clearly, the following briefly introduces the accompanying drawings required for describing the particular embodiments or the prior art. Apparently, the accompanying drawings in the following description show some embodiments of the present application. For a person skilled in the art, other drawings may also be obtained according to these drawings without creative efforts.
Fig. 1 is a graph of initial screening results of a methylation marker associated with endometrial cancer provided by an example of the present application;
Fig. 2 is a graph showing results of further screening for endometrial cancer methylation biomarkers based on high throughput sequencing data from cervical swabs in Chinese populations according to an example of the present application;
Fig. 3 shows results of diagnostic performance of the TXNRD1 gene in a sample;
Fig. 4 is a schematic flowchart of the methylation level detection of the TXNRD1 gene in a cervical exfoliated cell; and
Fig. 5 is a graph of ROC results after the detection of specific primer-probe combinations for 6 target regions of TXNRD1.

### Detailed Description of the Embodiments

Reference will now be provided in detail to embodiments of the present application, one or more examples of which are described below. Each example is provided by way of explanation and not limitation of the present application. Indeed, it will be apparent to a person skilled in the art that various modifications and variations can be made to the present application without departing from the scope or spirit of the present application. For example, features illustrated or described as part of one embodiment can be used in another embodiment to yield a still further embodiment.

Unless otherwise indicated, all terms (including technical and scientific terms) used to disclose the present application have the same meaning as commonly understood by one of ordinary skill in the art to which the present application belongs. The ensuing definitions serve to better understand the teachings of the present application by further guidance. The terminology used herein in the description of the present application is for the purpose of describing particular embodiments only and is not intended to be limiting of the present application.

As used herein, the term "and/or", "or/and", or "as well as/or" is inclusive of any one, and also inclusive of any and all combinations of two or more of the associated listed items, including any two, any more, or all combinations of the associated listed items. It should be noted that, when at least three items are connected with at least two conjunction combinations selected from the group consisting of "and/or", "or/and" and "as well as/or", it should be understood that, in the present application, the technical solution unambiguously contains the technical solution which is connected with the expression "logic and" and also undoubtedly contains the technical solution which is connected with the expression "logic or". For example, "A and/or B" includes three parallel solutions A, B, and A+B. For another example, the technical solutions "A, and/or, B, and/or, C, and/or, D" include any one of A, B, C, and D (i.e. the technical solutions which are all connected with "logic or"), and also include any and all combinations of A, B, C, and D, that is, include combinations of any two or three of A, B, C, and D, and further include a combination of the four, i.e. A, B, C and D (i.e. technical solution obtained by connecting the four by means of "logic and").

The terms "comprising", "containing", and "including" as used in the present application are synonymous, are inclusive or open-ended, and do not exclude additional, unreferenced members, elements, or method steps.

The recitation of numerical ranges by endpoints in the present application includes all numbers and fractions subsumed within that range, as well as the recited endpoints.

The present application relates to a concentration value, and the meaning thereof contains fluctuation within a certain range, for example, fluctuation within a corresponding accuracy range. For example, 2% may allow fluctuations within a range of ±0.1%. For values that are large or do not require too fine control, their meaning is allowed to include greater fluctuations. For example, 100 mM is allowed to include fluctuations in the range of ±1%, ±2%, ±5%, etc. In relation to molecular weight, a fluctuation of ±10% is allowed.

In the present application, descriptions such as "a plurality of" and "multiple" are involved, and if not particularly limited, it means that the number is greater than or equal to 2.

In the present application, the technical features described in the open-ended mode define a closed technical solution constituted by the listed features, and also define open technical solutions containing the listed features.

In the present application, "preferably", "more preferably", "even more preferably", and "preferred" are only used for describing embodiments or examples with better effects, and should be understood as not limiting the scope of protection of the present application. In the present application, "optionally", "optional", and "options" means that one element may be present or absent, that is, means that two parallel solutions which "have" or "do not have" the element can be selected. If a plurality of "optional" appears in a technical solution, and there is no particular description, and there is no contradiction or a mutual restriction relationship, each "optional" is independent.

As used herein, the term "diagnosis" refers to the act or process of identifying or determining a disease or disorder or a factor causing the disease or disorder in a mammal by evaluating signs and symptoms of the disease or disorder or by means of genetic analysis, pathological analysis, histological analysis, and the like; and the direct aim is to judge the presence or absence of the disease. Generally, a disease or disorder is diagnosed on the basis of evaluation of one or more factors and/or symptoms of the disease. That is, the diagnosis may be made according to the presence or absence of a factor symbolizing the presence or absence of a disease or disorder, or the amount thereof. Each factor or symptom considered to symbolize a particular disease for diagnosis need not be uniquely associated with the particular disease; That is, there may be different diagnoses that can be inferred from diagnostic factors or symptoms. Likewise, there may be instances where in an individual who does not have a particular disease, there are factors or symptoms symbolizing the particular disease.

A subject to be diagnosed may be a subject suspected of having endometrial cancer. "Subject suspected of having endometrial cancer" encompasses individuals who have received a preliminary diagnosis (e.g., a CT scan showing clumps, or increased levels of markers such as CA199 and CA125) and whose cancer stage has not yet become clear. The term also encompasses those who have suffered from endometrial cancer (e.g., individuals in remission). As used herein, the term "cancer stage" refers to a qualitative or quantitative assessment of the level of cancer development. Criteria for determining the cancer stage include, but are not limited to, the size of the tumor and the extent of metastasis (e.g., localized or distant). In some embodiments, "subject" is a control subject suspected of having endometrial cancer or diagnosed with endometrial cancer. In some embodiments, the diagnosis is early diagnosis.

As used herein, the primary goal of the term "screening" is to find an early disease or risk of disease in a large number of otherwise healthy persons with the aim of assessing the high or low risk of developing a certain health problem. In some embodiments, screening is early screening.

All documents mentioned in the present application are herein incorporated by reference as if the documents were each individually incorporated by reference. Unless otherwise conflicting with the purpose and/or technical solution of the present application, the cited documents mentioned in the present application are incorporated by reference in their entirety for all purposes. When the present application relates to a cited document, definitions of relevant technical features, terms, nouns, phrases, etc. in the cited document are also referred to. When the present application relates to a cited document, examples and preferences of the relevant technical features referred to are also incorporated by reference in the present application, but are not intended to limit the present application. It should be understood that when the cited content conflicts with the description in the present application, the present application shall prevail or the reference shall be modified adaptively based on the description in the present application.

A first aspect of the present application relates to a primer-probe combination product, comprising at least one of combinations a)-f):
a) primers having nucleotide sequences as shown in SEQ ID NOs: 1-2 and a probe having a nucleotide sequence as shown in SEQ ID NO: 3;
b) primers having nucleotide sequences as shown in SEQ ID NOs: 4-5 and a probe having a nucleotide sequence as shown in SEQ ID NO: 6;
c) primers having nucleotide sequences as shown in SEQ ID NOs: 7-8 and a probe having a nucleotide sequence as shown in SEQ ID NO: 9;
d) primers having nucleotide sequences as shown in SEQ ID NOs: 10-11 and a probe having a nucleotide sequence as shown in SEQ ID NO: 12;
e) primers having nucleotide sequences as shown in SEQ ID NOs: 13-14 and a probe having a nucleotide sequence as shown in SEQ ID NO: 15; and
f) primers having nucleotide sequences as shown in SEQ ID NOs: 16-17 and a probe having a nucleotide sequence as shown in SEQ ID NO: 18.

The primer-probe combination product provided in the present application is used for detecting the methylation of a chr12:104215491-104216453 fragment of the TXNRD1 gene, wherein after a sulfite treatment is performed on a sample nucleic acid, unmethylated cytosine (C) is deaminated to uracil (U), and methylated cytosine (5 mC) remains unchanged; and then, by fluorescence PCR amplification, uracil (U) is converted into thymine (T), and 5 mC is restored to C. The accumulation of fluorescence signals in the whole PCR process can be monitored in real time, and quantitative analysis is performed by comparing same with a standard curve.

The combination product provided by the present application has high sensitivity and good specificity.

In some embodiments, the combination product contains a primer and a probe for detecting an internal reference nucleic acid.

In some embodiments, the internal reference nucleic acid is a COL2A1 gene or a fragment thereof.

In addition to the COL2A1 gene, other internal reference genes such as β-actin, GAPDH, B2M, SDHA, and HPRT1 for methylation detection in the prior art may also be used. The internal reference gene exists in both normal cells and cancer tissue cells, and does not have sequence diversity. Therefore, the detection sample can be better monitored in real time, and the methylation ratio of the target gene in the sample can be better reflected.

In some embodiments, the nucleotide sequence of the primer for detecting the internal reference nucleic acid is as shown in SEQ ID NOs: 19-20, and the nucleotide sequence of the probe for detecting the internal reference nucleic acid is as shown in SEQ ID NO: 21.

In addition, it should be noted that, in one aspect, useful primers and probes include nucleotide sequences having at least 80%, 85%, 90%, 91%, 92%, 93%, 94%, 95%, 96%, 97%, 98%, or 99% identity to the primers or probes set forth in SEQ ID NOs: 1-21. Such primer and probe modifications are also contemplated and may be prepared according to standard techniques.

The term "% identity", in the context of two or more nucleotide sequences or amino acid sequences, refers to two or more sequences or subsequences that are the same or have a specified percentage of identical amino acid residues or nucleotides, when compared and aligned for maximum correspondence, as measured using one of the following sequence comparison algorithms or by visual inspection. For example, % identity is relative to the entire length of the coding region of a sequence to be compared.

For sequence comparison, typically, one sequence is used as a reference sequence to which a test sequence is compared. When using a sequence comparison algorithm, the test and reference sequences are input into a computer, and if necessary, subsequence coordinates are specified, and sequence algorithm program parameters are specified. The sequence comparison algorithm then calculates the percent sequence identity of the test sequence to the reference sequence based on the specified program parameters. The percent identity can be determined using a search algorithm such as BLAST and PSI-BLAST (Altschul et al., 1990, J Mol Biol 215:3, 403-410; Altschul et al., 1997, Nucleic Acids Res 25:17, 3389-402).

Common general knowledge can be used in primer and probe modifications. Modified versions of these primer and/or probe sequences can contain, by way of non-limiting example, adding one or more nucleotides to the 5' end, adding one or more nucleotides to the 3' end, adding one or more nucleotides to the 5' end and the 3' end, adding tails, shortening the sequence, lengthening the sequence, shifting several bases upstream and downstream in the sequence, or any combination thereof.

Base modifications include 3'P, 5'P, 5-nitroindole, 2-aminopurine, 8-amino-2'-deoxyadenosine, C-5 propynyl-deoxycytidine, C-5 propynyl-deoxyuridine, 2-amino-2'-deoxyadenosine-5'-triphosphate, 2,6-diaminopurine (2-amino-dA), reverse dT, reverse dideoxy-T, hydroxymethyl dC, iso-dC, 5-methyl dC, aminoethyl-phenoxazine-deoxycytidine, and locked nucleic acids (LNA's), and contain at least one mismatched base at one of the bases, or at least one RNA base substitution, to achieve, for example, the increase of nucleic acid interactions at the 3' end of the mutant specific primer or the increase of Tm (Melting temperature). The addition of a double-stranded stable base modification has a positive effect on PCR, so that it can be performed at a relatively high temperature, and in this range, it is known that Taq polymerase shows the maximum activity. The modified probe should retain the ability to distinguish a mutation site to be detected from a wild type site.

In some embodiments, the probe is labeled with a detectable signal substance. In some embodiments, the signal substances are fluorophores, colorimetric labels, colloidal gold, quantum dots, biotin and other label molecules that can be used for detection (e.g., alkyne groups for Raman diffraction imaging, cycloalkenes for the click reaction, and initiating groups for polymer labels), and can also be selected from polypeptide/protein molecules, LNA/PNA, unnatural amino acids and analogs thereof (such as peptidomimetics), unnatural nucleic acids and analogs thereof (nucleotide mimetics), and nanostructures (including inorganic nanoparticles, NV-center, aggregation/assembly inducing luminescent molecules, rare earth ion ligand molecules, multimetal oxygen clusters, etc.).

In some embodiments, the probes are self-quenching probes.

In some embodiments, the fluorescent emitting groups of the probes are each independently any one selected from AMCA, Pacific Blue, Atto 425, BODIPY FL, FAM, Alexa Fluor 488, TET, JOE, Yakima Yellow, VIC, HEX, Quasar 570, Cy3, NED, TAMRA, ROX, Aqua Phluor593, Texas Red, Atto 590, Cy5, Quasar 670, and Cy5.5.

In some embodiments, quenching groups of the probes are each independently any one selected from BHQ1, BHQ2, BHQ3, Dabcyl, Eclipse, and MGB.

A second aspect of the present application also relates to a kit comprising the primer-probe combination product described above.

The term "kit" refers to any article (e.g., a package or container) that contains at least one device, and the kit may further contain an instruction for use, a supplemental reagent, and/or a component or an assembly components used in the method or steps thereof described herein.

In some embodiments, the kit further contains at least one of an amplification buffer, dNTP, Mg²⁺, a DNA polymerase, a positive control, a negative control, water, and a bisulfite sequencing reagent.

In some embodiments, the DNA polymerase has 5' exonuclease activity, e.g., any one selected from the group consisting of Taq, Bst, Vent, Phi29, Pfu, Tru, Tth, Tl1, Tac, Tne, Tma, Tih, Tf1, Pwo, Kod, Sac, Sso, Poc, Pab, Mth, Pho, ES4 DNA polymerase, and Klenow fragment.

In some embodiments, the water is generally nucleic acid- and/or nuclease-free water. The water may be distilled water, deionized water, or reverse osmosis water.

In some embodiments, the positive sample contains a methylated target; and it may be a plasmid containing fragment sequences detected by the combinations a)-f).

In some embodiments, the negative sample is human genomic DNA without target gene methylation, or purified water.

In some embodiments, the bisulfite sequencing reagent contains sulfite.

The primer or a set of primers provided by the present application need to be used in conjunction with the bisulfite sequencing method.

Bisulfite sequencing method is currently the most widely used method for methylation detection. Its principle is that the bisulfite treatment converts unmethylated C (cytosine) into U (uracil), which is transformed into T after a PCR process, while methylated C will not change after the bisulfite treatment.

In some embodiments, the bisulfite sequencing reagent further contains an exogenous DNA not associated with a nucleic acid fragment to be detected; further, the exogenous DNA is a λDNA without a methylation modification.

The nucleic acid fragment to be detected is preferably treated together with the exogenous DNA (in particular, the λDNA without a methylation modification) with bisulfite to convert unmethylated cytosine into uracil. The effect of the exogenous DNA is to perform high-efficiency co-treatment with a sample during bisulfite treatment, and to play a protective role on a trace amount of DNA fragments, thereby minimizing the damage of bisulfite to trace amount of DNA.

The components are preferably achieved in the form of lyophilizates, e.g. in the form of one or more so-called lyophilized beads. Lyophilized beads may generally be understood to mean lyophilizates which are compressed into a spherical form after manufacture, after which the substances are usually present as powder. Therefore, components, in particular a DNA polymerase, a nucleic acid component and a reaction buffer component, required for a PCR batch may be provided, for example, in lyophilized forms. In this way, the PCR process can be started directly in a very user-friendly manner by adding the sample to be quantified and optionally other required components. In particular, the provision of lyophilized forms is very advantageous for automated applications.

A third aspect of the present application relates to use of a detection agent for the methylation level of TXNRD1 gene in the preparation of an endometrial cancer diagnosis and/or screening reagent or kit.

In some embodiments, the detection agent is used for detecting the methylation level of a chr12:104215491-104216453 fragment of the TXNRD1 gene.

In some embodiments, the detection agent is used for detecting the methylation level of at least one of the following regions:
chr12:104215491-104215646, chr12:104215647-104215765, chr12:104215750-104215872, chr12:104215852-104216065, chr12:104216066-104216200, and chr12:104216190-104216453.

In some embodiments, the detection agent is used for achieving any one or more of the methods:
methylation-specific PCR (such as digital PCR and quantitative methylation specific PCR), pyrosequencing or bisulfite sequencing, methylation-specific microarray, methylation-specific high performance liquid chromatography, high resolution melting (HRM) analysis, methylation-sensitive restriction endonuclease or fluorescence quantitation, methylation-specific denaturation high performance liquid chromatography, HPLC-UV, LC-MS/MS, methylation-sensitive single-strand conformation analysis, methylation-sensitive denaturation gradient gel electrophoresis, methylation-sensitive melting curve analysis, Methylight technology, methylation-sensitive dot blot analysis, enrichment methods of methylated antibody or MBD affinity, MeDIP-Seq, RRBS-Seq, WGBS, MBD-Seq, and SMRT.

The degree of methylation is preferably determined by means of PCR methods, for example, assay methods such as "fluorescence-based real-time PCR technology" (Eads et al., Cancer Res. 59: 2302-2306, 1999), Ms-SNuPE TM (methylation-sensitive single nucleotide primer extension) reaction (Gonzalgo & Jones, Nucleic Acids Res. 25: 2529-2531, 1997), methylation-specific PCR ("MSP"; Herman et al., Proc. Natl. Acad. Sci. USA 93:9821-9826, 1996; US Patent 5,786,146), and methylated CpG island amplification ("MCA"; Toyota et al., Cancer Res. 59: 2307-12, 1999) are used for determining the methylation level of TXNRD1 gene.

In some embodiments, the detection agent contains the primer-probe combination product described in the first aspect.

In some embodiments, the sample detected by the diagnosis and/or screening reagent or kit is a cervical exfoliated tissue or cell or a lysate thereof of a subject.

As used herein, "lysate" refers to a sample and/or biological sample material containing a lysed tissue or cell, that is, the structural integrity of the cervical exfoliated tissue or cervical exfoliated cell has been disrupted. To release the content of a cell or tissue sample, the material is typically treated with an enzyme and/or a chemical agent to dissolve, degrade, or disrupt the cell wall and cell membrane of such tissue or cell. A person skilled in the art is well familiar with appropriate methods for obtaining a lysate. This process is encompassed by the term "lysis".

The subject as referred to in the present application is a mammal, preferably a primate, and more preferably a human.

The present application also relates to a method for diagnosing and/or screening endometrial cancer, comprising:
amplifying a DNA sample which has been converted by means of a sulfite with the primer-probe combination described in the first aspect.

In some embodiments, the DNA sample is a genomic DNA.

In some embodiments, the DNA sample is from a cervical exfoliated tissue or cell or a lysate thereof.

For the results to be valid, the internal control channel has an S-shaped amplification curve and the Ct value is ≤30.

The Ct value of each methylated gene is recorded separately for performance evaluation, with Ct value less than 38 defined as positive.

In the present application, endometrial cancer may contain type I and/or type II endometrial cancer.

Embodiments of the present application will now be described in detail with reference to the following examples. It should be understood that these examples are intended to illustrate the present application only and are not intended to limit the scope of the present application. The following examples do not specify specific conditions of experimental methods. Reference is preferably made to the guidance given in the present application, and is also made to manual or conventional conditions in the art, other experimental methods known in the art, or conditions suggested by the manufacturer.

In the following specific examples, the measured parameters of raw material components may have slight deviations within the range of weighing accuracy unless otherwise specified. For temperature and time parameters, acceptable deviations caused by instrument testing accuracy or operational accuracy are allowed.

### Example 1 Screening of methylation biomarker for early screening of endometrial cancer

Firstly, with public database data, methylation biomarkers specific for endometrial cancer were preliminarily screened out. Then, the preliminarily screened methylation biomarkers were verified by performing high-throughput capture sequencing on cervical swab samples from the Chinese population to further identify better biomarkers.

### 1. Collection of data related to endometrial cancer in a public database

TCGA (The cancer genome atlas) is a joint project launched by National Cancer Institute (NCI) and National Human Genome Research Institute (NHGRI) in 2006, and collected clinical data, including genomic variation, mRNA expression, miRNA expression, methylation data, and other data, which are valuable data source for cancer researchers. The overall data quality on TCGA is high and the sample size is large. Therefore, this study used the methylation data of endometrial cancer in TCGA as the main analysis data source. Finally, 436 endometrial cancer tissue samples, 97 normal endometrial tissue samples, 20 normal cervical tissues, and 102 normal cervical swabs were collected.

2. Primary screening of methylation biomarker of endometrial cancer according to public database

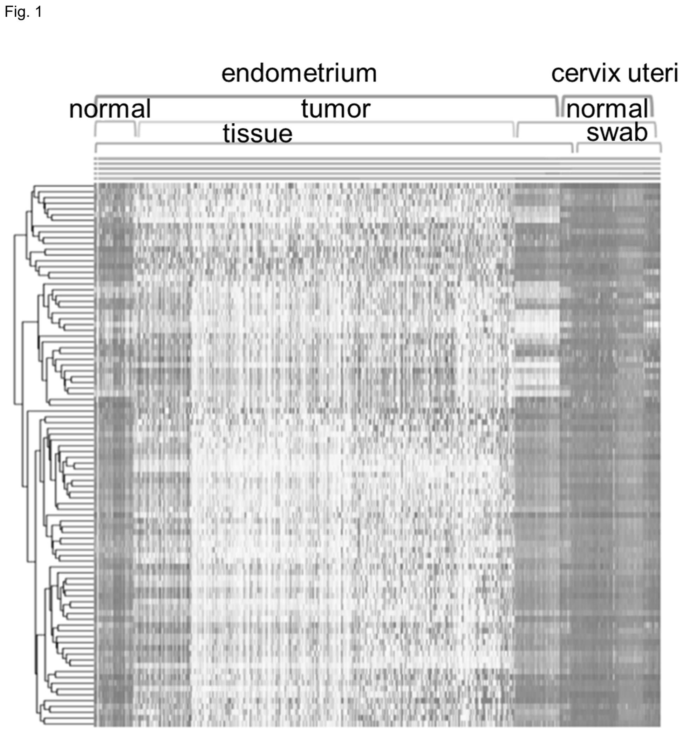

On the basis of the described collected public data, a preliminary screening of endometrial cancer methylation biomarker was performed. Specifically, two rounds of screening were performed in total. The first round of screening was primarily based on data from normal and tumor endometrial tissue. The screening was achieved based on a threshold for the difference in average methylation levels between endometrial tumor and normal tissue, as well as a threshold for the average number of CpG sites within a 100 bp interval. Following these criteria, a total of 232 biomarkers were obtained. The second round of screening was primarily based on normal cervical swabs. While meeting the described conditions, the average methylation level of normal cervical swabs also needed to meet specific thresholds. This resulted in a total of 94 biomarkers obtained. Specific results were shown in Fig. 1.

### 3. Further screening of endometrial cancer methylation biomarker according to high throughput sequencing data of cervical swabs of Chinese population

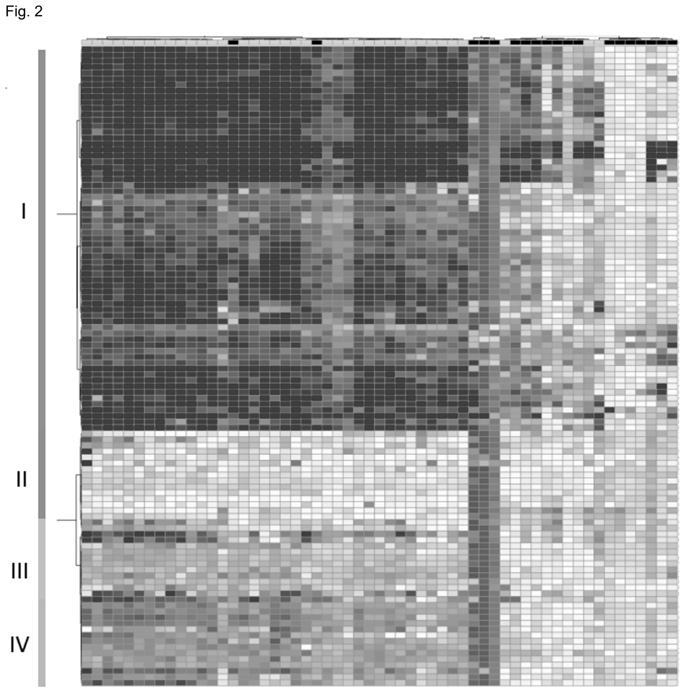

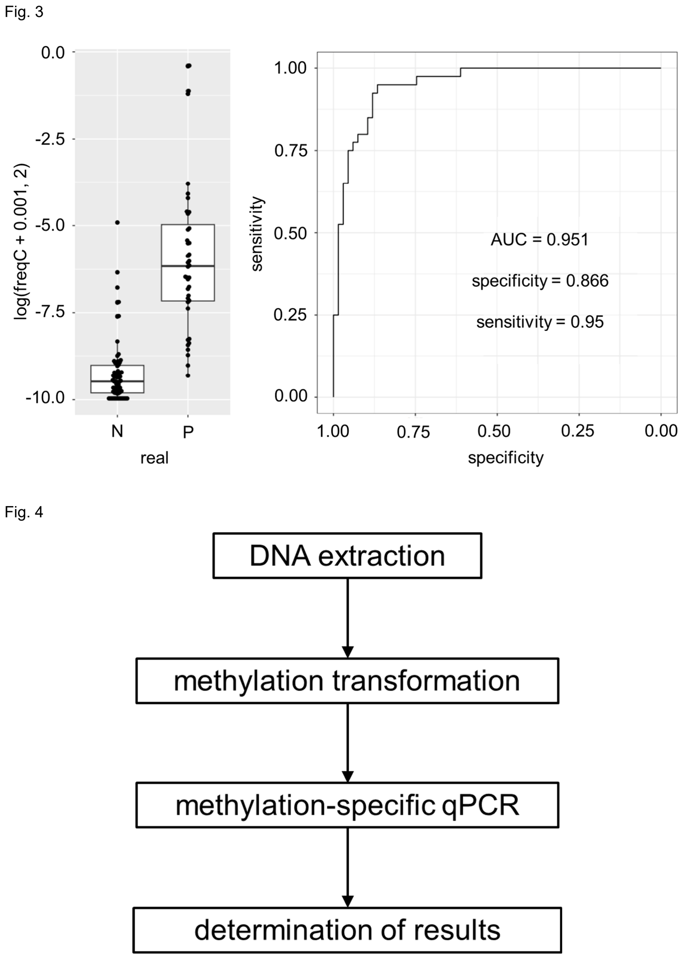

A total of 87 cervical swab samples were collected clinically from a Chinese population, including 28 endometrial cancer samples and 59 normal endometrial samples. High-throughput capture sequencing was performed to determine the methylation levels of the 94 biomarkers described above. Overall, all 94 biomarkers showed differences between tumor and normal samples. Using a clustering algorithm, these 94 biomarkers were grouped into four categories, wherein the first category had very low background methylation, but a large proportion of tumor samples had low methylation, making detection difficult. The second category had very high background methylation levels, resulting in excessive noise and hindering detection. And the third and fourth categories performed well, with the fourth category performing particularly well, having relatively low background and high methylation signals in tumor samples, as shown in Fig. 2. Ultimately, using the data from high-throughput capture sequencing of cervical swab samples from a Chinese population, we further selected 10 high-performance endometrial cancer methylation biomarkers. The TXNRD1 gene showed particularly strong performance across samples, as shown in Fig. 3.

### Example 2 Detection of TXNRD1 Gene

The present application found that using the TXNRD1 gene as a target and detecting the methylation status of the gene in cervical exfoliated cells can achieve early screening and diagnosis of endometrial cancer, and this method can avoid invasive sampling.

A total of 63 endometrial cancer specimens and 117 benign endometrial specimens with known pathological results were collected, all are cervical exfoliated cell samples. Specific primer and probe combinations targeting six target regions of TXNRD1 were tested, and the sensitivity, specificity, and ROC of these six regions were analyzed.

Specific detection systems and detection methods were shown below.

This example was directed to the TXNRD1 gene target region being chr12:104215491-104216453 (GRCh38/hg38 version). Primer and probe sequences were shown in Table 1, respectively:

**Table 1**

| No. | Target region | Primer or probe sequence | Sequence (5'-3') | SEQ ID NO: |
|---|---|---|---|---|
| | | forward primer | TTTTTTTTAATAGGAAAGAACGGTC | 1 |
| 1 | chr12:104215491-104215646 | reverse primer | GATCAATCCTCCCACCTCG | 2 |
| | | probe | TCGGTTGGGCGCGGTG | 3 |
| 2 | chr12:104215647-104215765 | forward primer | ACGTTTGGCGGAGGTCGC | 4 |
| | | reverse primer | CTTCGCTATTATCACCGAACG | 5 |
| | | probe | TTGTCGGTCGCGGGT | 6 |
| 3 | chr12:104215750-104215872 | forward primer | AGTTTCGTTGGGGCGGTC | 7 |
| | | reverse primer | CAAACGTCCTTCGACTCCG | 8 |
| | | probe | TTCGGCGTAGTTTATGTCGT | 9 |
| 4 | chr12:104215852-104216065 | forward primer | CGGTCGGAAGGTTCGTTTC | 10 |
| | | reverse primer | AAAACGACGATAACCGCCG | 11 |
| | | probe | TTTTCGTTTTCGGTCGTATT | 12 |
| 5 | chr12:104216066-104216200 | forward primer | CGAGATTTCGGTTCGGTTTTC | 13 |
| | | reverse primer | CGAAAAACGAAAACCGACG | 14 |
| | | probe | CTACCCCGCCCGCTAA | 15 |
| 6 | chr12:104216190-104216453 | forward primer | GGTATTTTCGGATTCGGTTGTC | 16 |
| | | reverse primer | AACGACCCGCCTAATTTCG | 17 |
| | | probe | ACCCGTTAAAACGTCCT | 18 |

| | | | | |
|---|---|---|---|---|
| Note: The 5' end of the probe was labeled with a fluorescent group, and the 3' end was labeled with a quenching group. | | | | |

The target region of the reference gene COL2A1 of the present application was chr12: 47987446-47987534
Forward primer: GGGAAGATGGGATAGAAGGGAATAT (SEQ ID NO: 19)
Reverse primer: AACAATTATAAACTCCAACCAC (SEQ **ID** NO: 20)
Probe: TTCATTCTAACCCAATACCT (SEQ ID NO: 21, the 5' end of the probe being labeled with a fluorescent group, and the 3' end being labeled with a quenching group)

A schematic diagram of the experimental procedure was shown in Fig. 4, including: cervical exfoliated cell DNA extraction, methylation transformation treatment, qPCR, etc.

### I. Cervical exfoliated cell DNA extraction

QIAamp^{®} DNA Mini Kit nucleic acid extraction reagent was employed as cell genomic DNA extraction reagent.

A preservation tube was centrifuged at 3,000 g for 1 minute.
1.1. Used a pipette to remove 300 µL of a sample storage solution (leaving approximately 300 µL of the storage solution in a storage tube) and transfered it to a 1.5 mL microcentrifuge tube.
1.2. Added 20 µL Proteinase K to the sample storage tube containing a sampling brush and mixed the sample and a reagent solution thoroughly in a shaker mixer.
1.3. Added 270 µL Buffer AL to the sample storage tube containing the sampling brush, placed the sample storage tube into a shaker mixer to thoroughly mix the sample with the reagent solution.
1.4. Placed the sample storage tube containing the sampling brush into a water bath or a metal bath at 56°C and heated for 1-2 hours, so that the sample is cracked, wherein during the heating, the sample storage tube can be irregularly placed in a shaking mixer for slight mixing, and then placed back for continued heating.
1.5. After the heating had been completed, removed the sample storage tube, placed same in a shaker mixer, and then maintained same at room temperature for cooling.
1.6. Added 400 µL of 100% alcohol, mixed same by shaking for 15 seconds.
1.7. Added all samples to QIAamp Mini Spin Column (with 2 mL collection tube), centrifuged at 10000 rpm for 1 minute, and discarded waste fluid and the collection tube (a maximum of 690 µL can be added each time, and if the sample volume was too large, it can be added in multiple times).
1.8. Inserted a spin column into a new collection tube, added 500 µL of Buffer AW1, reacted for 2 minutes, centrifuged at a rotation speed of 10000 rpm for 1 minute, and after centrifugation, discarded waste liquid and the collection tube.
1.9. Inserted a spin column into a new collection tube, add 500 µL of Buffer AW2, reacted for 2 minutes, centrifuged at a rotation speed of 10000 rpm for 1 minute, and after centrifugation, discarded waste liquid and the collection tube.
1.10. Re-inserted the spin column into the collection tube, centrifuged at a rotating speed of 12000 rpm for 3 minutes so as to facilitate the removal of residual alcohol, and discarded waste liquid and the collection tube after centrifugation.
1.11. Inserted the spin column into a new 1.5 mL microcentrifuge tube (indicating the serial number of the sample, the date and the DNA), dried for 5 min, added 60 µL of Buffer AE (the amount of Buffer AE can be increased appropriately according to the sample concentration, and the amount of Buffer AE was recorded), and placed the microcentrifuge tube for a reaction at room temperature for 10 minutes.
1.12. Centrifuged for 1 minute at 10000 rpm, and extracted the sample DNA.

### II. DNA bisulfite conversion

The extracted DNA was subjected to bisulfite conversion using the EZ DNA Methylation-Gold^{™} Kit as a DNA bisulfite conversion reagent. Unmethylated cytosine (C) in the DNA was converted to uracil (U), while methylated cytosine (C) remained unchanged, resulting in converted bis-DNA.

### 2. Bisulfite conversion:

2.1. When the total amount of DNA was calculated to be 320 ng, calculated the required DNA volume, and pipetted the calculated DNA sample volume to a new PCR tube.
2.2. Added suitable NFW (nuclease free water) for dilution, brought the diluted sample to a total volume of 20 µL, and labeled the sample number.
2.3. Respectively added 130 µL of CT Conversion Reagent into the samples of DNA that had been diluted to a total volume of 20 µL, and placed the samples in a shaker mixer for brief centrifugation.
2.4. Placed the sample tube in a PCR instrument with the PCR program settings shown in Table 2 below.

**Table 2**

| Procedure Name | Set temperature (°C) | Time |
|---|---|---|
| Denaturation | 98 | 10 minutes |
| Heat preservation | 64 | 2.5 hours |
| Cooling | 4 | ∞ |

2.5. After the reaction had been completed, prepared equal numbers of Zymo-Spin^{™} IC Column and 1.5 mL microcentrifuge tubes according to the amount of reaction required for experiments, and pre-labeled sample numbers on the Zymo-Spin^{™} IC Column lid, collection tube, and 1.5 mL microcentrifuge tube.
2.6. Added 600 µL of M-Binding Buffer in Zymo-Spin^{™} IC Column.
2.7. Added all the sample in the sample tube to the Zymo-Spin^{™} IC Column, and mixed thoroughly by shaking the column. Made sure the sample number is the same.
2.8. Placed the Zymo-Spin^{™} IC Column in a high-speed micro centrifuge, and centrifuged at 10,000 rpm for 1 minute.
2.9. After the lower collection tube waste liquid had been decanted, carefully reloaded the Spin Column, and added 100 µL of M-Wash Buffer to the Spin Column.
2.10. After 2 minutes of reaction, centrifuged the mixture at 10,000 rpm for 1 minute, removed the Zymo-Spin^{™} IC Column from the centrifuge, and placed same in a microcentrifuge tube holder.
2.11. Opened the lid, added 200 µL of M-Desulphonation Buffer for reaction at room temperature for 15 minutes, and then centrifuged at 10,000 rpm for 1 minute.
2.12. Removed Zymo-Spin^{™} IC Column from the centrifuge, opened the lid, added 200 µL of M-Wash Buffer, and reacted for 2 minutes, followed by centrifugation at 10,000 rpm for 1 minute.
2.13. Repeated the previous step, removed the Zymo-Spin^{™} IC Column from the centrifuge, opened the lid, added 200 µL of M-Wash Buffer, and reacted for 2 minutes, followed by centrifugation at 10,000 rpm for 1 minute.
2.14. After the lower collection tube waste liquid had been decanted, carefully reloaded the Spin Column, and centrifuged at 12,000 rpm for 3 minutes so as to facilitate the removal of residual alcohol.
2.15. Inserted the upper Spin Column into a pre-prepared 1.5 mL microcentrifuge tube, took care to confirm whether the sample number was the same as its corresponding Spin Column, opened the upper Spin Column lid, and left at room temperature for 5 minutes until alcohol was evaporated completely.
2.16. Added 40 µL of M-Elution buffer to the film of Spin Column, closed the Spin Column lid, stood and reacted at room temperature for 10 minutes. After the reaction had been completed, centrifuged at 10,000 rpm for 1 minute to extract BS-DNA (DNA after conversion by bisulfite).
2.17. Collected the extracted DNA from the lower microcentrifuge tube, re-confirmed if the microcentrifuge tube sample number was consistent with the number on Spin Column, and after confirmation, marked the sample status as BS-DNA.
2.18. The collected BS-DNA can be directly subjected to subsequent methylation-specific qPCR detection and can also be temporarily stored at -20°C or -80°C.

### 3. Methylation-specific qPCR

3.1. The PCR reaction solution and the primer-probe mixture were as shown in Table 3:

**Table 3**

| Component | Amount per portion (µL) |
|---|---|
| Forward primer (10 µM) | 1 |
| Reverse primer (10 µM) | 1 |
| Probe (10 µM) | 0.5 |
| qPCR master mix | 10 |
| Nuclease-free water | 3.5 |
| DNA after methylation conversion | 4 |
| Formulated total volume per portion | 20 |

3.2. PCR reaction conditions

Pre-denaturation at 95°C for 5 min; denaturation at 95°C for 15 s, and annealing extension at 60°C for 35 s, 50 cycles; and holding at 25°C for 10 min.

Signal collection: collection of fluorescence signals at 60°C.
3.3. Interpretation of Results
3.3.1 The results were valid if the internal standard channel had an S-shaped amplification curve and the Ct value was ≤30.
3.3.2 The Ct value of each methylated gene was recorded for performance evaluation, and a Ct value below 38 was defined as positive.

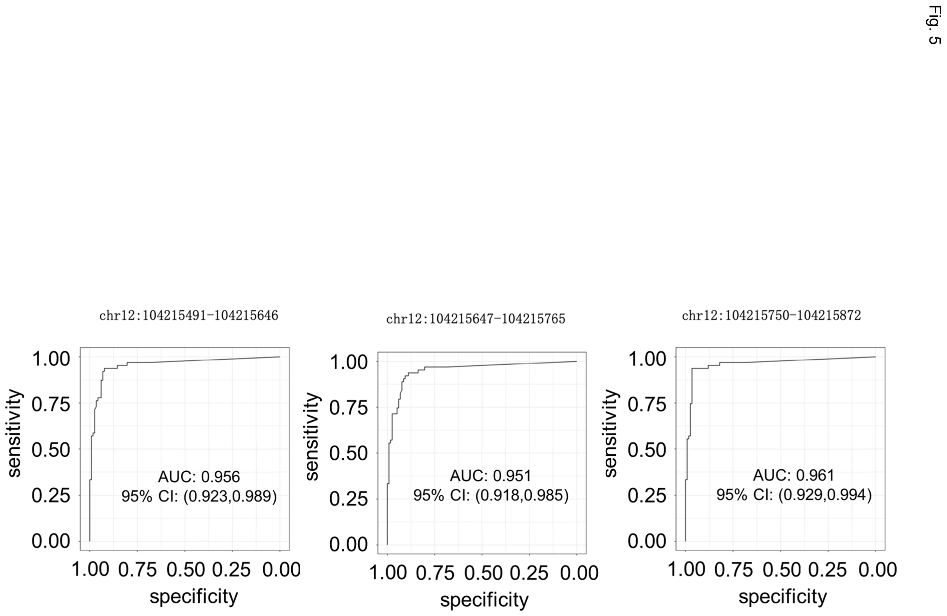

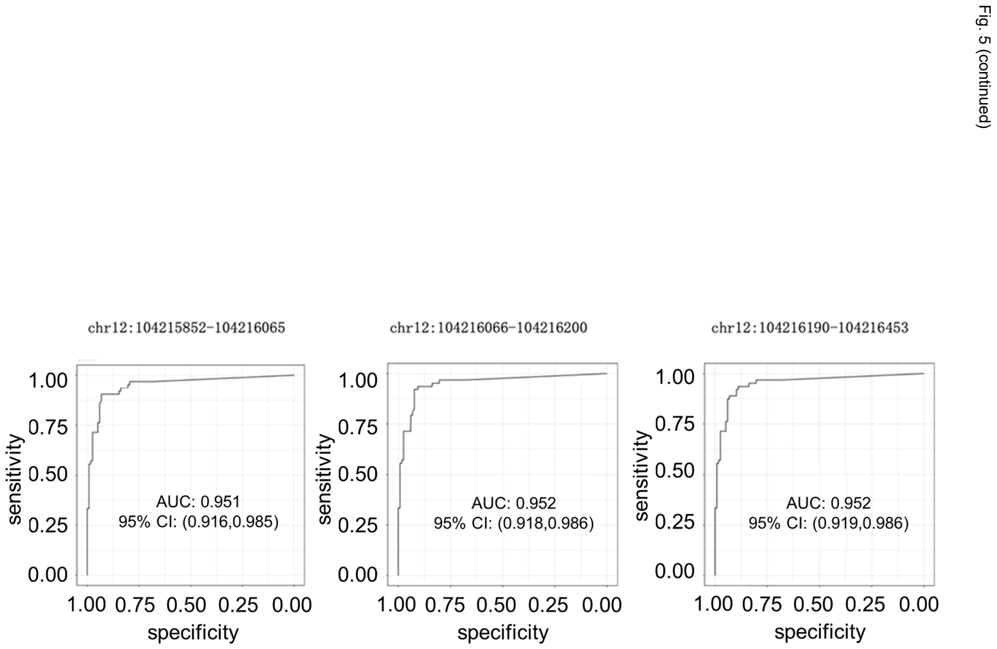

The detection results were shown in Fig. 5 and Table 4 below.

**Table 4**

| TXNRD1 methylated region | True negative/false positive | True positive/false negative | Specificity | Sensitivity | AUC (area under ROC curve) |
|---|---|---|---|---|---|
| chr12:104215491-104215646 | 108/9 | 56/7 | 92.3% | 88.9% | 0.956 |
| chr12:104215647-104215765 | 107/10 | 57/6 | 91.4% | 90.47% | 0.951 |
| chr12:104215750-104215872 | 112/5 | 59/4 | 96.6% | 93.7% | 0.962 |
| chr12:104215852-104216065 | 109/8 | 57/6 | 93.2% | 90.47% | 0.95 |
| chr12:104216066-104216200 | 108/9 | 58/5 | 92.3% | 92.1% | 0.951 |
| chr12:104216190-104216453 | 109/8 | 56/7 | 93.2% | 88.9% | 0.952 |

The results indicated that the sensitivity and specificity of detection of endometrial cancer with 6 methylation regions of TXNRD1 based on cervical exfoliated cell samples were greater than 88% and 91%, respectively, and that the area under the ROC curve was greater than 0.95. The detection effect of chr12:104215750-104215872 region was the best, the sensitivity and specificity were 93.7% and 96.6%, respectively, and the area under the ROC curve was 0.961.

The foregoing examples merely represent several embodiments of the present application, and are described in detail, but are not intended to limit the scope of the present application. It should be noted that, for a person skilled in the art, various modifications and improvements can be made without departing from the concept of the present application, and all these modifications and improvements belong to the scope of protection of the present application. Therefore, the scope of protection of the present application should be subject to the appended claims, and the description and drawings can be used to interpret the contents of the claims.

## Claims

1. A primer-probe combination product, comprising at least one group of combinations a)-f):
a) primers having nucleotide sequences as shown in SEQ ID NOs: 1-2 and a probe having a nucleotide sequence as shown in SEQ ID NO: 3;
b) primers having nucleotide sequences as shown in SEQ ID NOs: 4-5 and a probe having a nucleotide sequence as shown in SEQ ID NO: 6;
c) primers having nucleotide sequences as shown in SEQ ID NOs: 7-8 and a probe having a nucleotide sequence as shown in SEQ ID NO: 9;
d) primers having nucleotide sequences as shown in SEQ ID NOs: 10-11 and a probe having a nucleotide sequence as shown in SEQ ID NO: 12;
e) primers having nucleotide sequences as shown in SEQ ID NOs: 13-14 and a probe having a nucleotide sequence as shown in SEQ ID NO: 15; and
f) primers having nucleotide sequences as shown in SEQ ID NOs: 16-17 and a probe having a nucleotide sequence as shown in SEQ ID NO: 18.

2. The combination product according to claim 1, further comprising a primer and a probe for detecting an internal reference nucleic acid.

3. The combination product according to claim 2, wherein the internal reference nucleic acid is a COL2A1 gene or a fragment thereof.

4. The combination product according to claim 3, wherein nucleotide sequences of primers for detecting the internal reference nucleic acid are as shown in SEQ ID NOs: 19-20, and a nucleotide sequence of a probe for detecting the internal reference nucleic acid is as shown in SEQ ID NO: 21.

5. The combination product according to any one of claims 1-4, wherein the probes are all self-quenching probes.

6. The combination product of claim 5, wherein fluorescent emitting groups of the probes are each independently any one selected from AMCA, Pacific Blue, Atto 425, BODIPY FL, FAM, Alexa Fluor 488, TET, JOE, Yakima Yellow, VIC, HEX, Quasar 570, Cy3, NED, TAMRA, ROX, Aqua Phluor593, Texas Red, Atto 590, Cy5, Quasar 670, Cy5.5, and Cy5.5.

7. The combination product according to claim 5, wherein quenching groups of the probes are each independently any one selected from BHQ1, BHQ2, BHQ3, Dabcyl, Eclipse, and MGB.

8. A kit, comprising the primer-probe combination product according to any one of claims 1-7.

9. The kit according to claim 8, further comprising at least one of an amplification buffer, dNTP, Mg²⁺, a DNA polymerase, a positive control, a negative control, water, and a bisulfite sequencing reagent.

10. Use of a detection agent for the methylation level of TXNRD1 genein the preparation of an endometrial cancer diagnosis and/or screening reagent or kit.

11. The use according to claim 10, wherein the detection agent is used for detecting the methylation level of a chr12:104215491-104216453 region of the TXNRD1 gene.

12. The use according to claim 11, wherein the detection agent is used for detecting the methylation level of at least one of the following regions:
chr12:104215491-104215646, chr12:104215647-104215765, chr12:104215750-104215872, chr12:104215852-104216065, chr12:104216066-104216200 and chr12:104216190-104216453.

13. The use according to any one of claims 10-12, wherein the detection agent is used for implementing any one or more of the following methods:
methylation-specific PCR, pyrosequencing or bisulfite sequencing, methylation-specific microarray, methylation-specific high performance liquid chromatography, high resolution melting (HRM) analysis, methylation-sensitive restriction endonuclease or fluorescence quantitation, methylation-specific denaturation high performance liquid chromatography, HPLC-UV, LC-MS/MS, methylation-sensitive single-strand conformation analysis, methylation-sensitive denaturation gradient gel electrophoresis, methylation-sensitive melting curve analysis, Methylight technology, methylation-sensitive dot blot analysis, enrichment methods of methylated antibody or MBD affinity, MeDIP-Seq, RRBS-Seq, WGBS, MBD-Seq, and SMRT.

14. The use according to claim 11, wherein the detection agent comprises the primer and probe combined product according to any one of claims 1-7.

15. The use according to any one of claims 10-12, wherein a sample to be detected by the diagnosis and/or screening agent or kit is a cervical exfoliated tissue or cell or a lysate thereof of the subject.

16. A method for diagnosing and/or screening endometrial cancer, wherein the method comprises: realizing the diagnosis and/or screening of the endometrial cancer by detecting the methylation level of TXNRD1 gene.

17. The method of claim 16, wherein detecting the methylation level of the TXNRD1 gene comprises detecting the methylation level of the chr12:104215491-104216453 region of the TXNRD1 gene.

18. The method according to claim 17, wherein detecting the methylation level of the TXNRD1 gene comprises detecting the methylation level of at least one of the following regions:
chr12:104215491-104215646, chr12:104215647-104215765, chr12:104215750-104215872, chr12:104215852-104216065, chr12:104216066-104216200, and chr12:104216190-104216453.

19. The method according to any one of claims 16-18, wherein the detection comprises any one or more of the following methods:
methylation-specific PCR, pyrosequencing or bisulfite sequencing, methylation-specific microarray, methylation-specific high performance liquid chromatography, high resolution melting (HRM) analysis, methylation-sensitive restriction endonuclease or fluorescence quantitation, methylation-specific denaturation high performance liquid chromatography, HPLC-UV, LC-MS/MS, methylation-sensitive single-strand conformation analysis, methylation-sensitive denaturation gradient gel electrophoresis, methylation-sensitive melting curve analysis, Methylight technology, methylation-sensitive dot blot analysis, enrichment methods of methylated antibody or MBD affinity, MeDIP-Seq, RRBS-Seq, WGBS, MBD-Seq, and SMRT.

20. The method according to claim 16, wherein the detection agent for the detection comprises the primer-probe combination product according to any one of claims 1-7.

21. The method according to any one of claims 16-18, wherein the sample to be detected in the method is a cervical exfoliated tissue or cell or a lysate thereof of the subject.

22. A primer-probe combination for diagnosing and/or screening endometrial cancer, wherein the primer-probe combination is used for detecting the methylation level of TXNRD1 gene.

23. The primer-probe combination of claim 22, wherein detecting the methylation level of the TXNRD1 gene comprises detecting the methylation level of a chr12:104215491-104216453 region of the TXNRD1 gene.

24. The primer-probe combination of claim 22, wherein detecting the methylation level of the TXNRD1 gene comprises detecting the methylation level of at least one of the following regions: chr12:104215491-104215646, chr12:104215647-104215765, chr12:104215750-104215872, chr12:104215852-104216065, chr12:104216066-104216200, and chr12:104216190-104216453.

25. The primer-probe combination according to any one of claims 22-24, wherein the detection comprises any one or more of the following methods:
methylation-specific PCR, pyrosequencing or bisulfite sequencing, methylation-specific microarray, methylation-specific high performance liquid chromatography, high resolution melting (HRM) analysis, methylation-sensitive restriction endonuclease or fluorescence quantitation, methylation-specific denaturation high performance liquid chromatography, HPLC-UV, LC-MS/MS, methylation-sensitive single-strand conformation analysis, methylation-sensitive denaturation gradient gel electrophoresis, methylation-sensitive melting curve analysis, Methylight technology, methylation-sensitive dot blot analysis, enrichment methods of methylated antibody or MBD affinity, MeDIP-Seq, RRBS-Seq, WGBS, MBD-Seq, and SMRT.

26. The primer-probe combination according to claim 22, wherein the primer-probe combination is the primer-probe combination product according to any one of claims 1-7.

27. The primer-probe combination of any one of claims 22-24, wherein the detection sample used for the detection is a cervical exfoliated tissue or cell or a lysate thereof of the subject.
